# EUROPEAN PATENT SPECIFICATION

(11) **EP 3 544 130 B1**
(45) Date of publication and mention of the grant of the patent: **27.01.2021**
(21) Application number: 18832452.9
(22) Date of filing: 23.03.2018
(51) Int. Cl.: H01T 23/00, A61L 9/22

(54) **BIPOLAR ION GENERATOR FOR AIR PURIFICATION AND DIFFUSER USING BIPOLAR ION GENERATOR**
BIPOLARER IONENGENERATOR ZUR LUFTREINIGUNG UND DIFFUSOR MIT BIPOLAREM IONENGENERATOR
GÉNÉRATEUR D'IONS BIPOLAIRE SERVANT À LA PURIFICATION DE L'AIR ET DIFFUSEUR D'AIR FAISANT APPEL AU GÉNÉRATEUR D'IONS BIPOLAIRE

(30) Priority: 11.07.2017 CN 201710559917
(43) Date of publication of application: 25.09.2019
(73) Proprietor: Shenzhen Yuan Qi Environmental Energy Technology Co., Ltd., Shenzhen, Guangdong 518048 (CN)
(72) Inventor: NI, Yunshi, Shenzhen, Guangdong 518048 (CN)
(74) Representative: Gulde & Partner
(86) International application number: PCT/CN2018/080245
(87) International publication number: WO 2019/011002

(56) References cited:
- WO-A1-2016/121153
- CN-A- 1 551 432
- CN-A- 107 453 214
- CN-Y- 2 277 788
- JP-A- 2002 065 838
- TW-A- 201 104 192
- US-A1- 2004 253 417
- US-A1- 2011 216 467

## Description

### FIELD OF THE INVENTION

The present invention relates to an ionizer, in particular to an alternating bipolar ionizer, and in particular to an alternating bipolar ionizer for air purification.

### BACKGROUD OF THE INVENTION

In the prior art, the following structures are mainly used to purify air by gas ionization.
1. Needle tip discharge device (unipolar). Needle tip discharge is the most familiar gas discharge device. Because the needle tip has a small area and a large curvature change, it is the easiest to discharge among the same kind of materials, and therefore it is the mostly and earliest used in the field of electrostatic precipitation. The needle tip was placed in a hexagonal honeycomb array structure in an early stage. Later, it is changed into a round hole structure with a tip, which has obvious effects on dust collection. Because it is unipolar and does not have redox capability, it does not have the function of eliminating odor. As a unipolar generator, whether it is a positive or negative one, it cannot meet the needs of improving air quality. In recent years, carbon fiber bundles have also been used as discharge electrodes. In terms of consistency, they are superior to needle tips, but bundle effects can cause gelation and failure. So is it possible to achieve positive and negative ion excitation on a single needle tip. Experiments have shown that due to the high voltage of AC, a sharp oxidation phenomenon occurs at the needle tip, which quickly blunts the needle tip, making it impossible to continue to discharge and thus causing failure. Therefore, it is difficult to generate bipolar ions in a single needle tip device under normal conditions.
2. A device using a wire to discharge (unipolar). A device that uses a wire (generally a tungsten wire) to discharge generally includes a pair of high voltage (typical power supply configuration is +8150V) tungsten wires, a separator which is located between the high voltage wires and is grounded, and a dust collecting plate (-3650V). This configuration is a unipolar, air-purifying module with electrostatic dust collection as the main function. Due to the uniformity of the diameter of the tungsten wire used, it is impossible to result in rapid passivation by concentrated discharging point. Particles contained in air flow passing through a high voltage generator will be positively charged. It is easy for the particles to be retained in a surface of negatively polarized dust collecting plate when reaching the same plate. This structure is effective. However, it does not have the mechanism for degradation and oxidation of the odor generated by indoor organic volatiles. Therefore, in this type of device, a negative ionizer (generating a small amount of ozone) is often added to a back end to make up for its deficiency.
   The above two forms of discharge have a very effective function, which is the visual effect of crushing the aerosol in an instant. Many manufacturers use it as a demonstrator to allow the soot to flow through the generator naturally when thermodynamic expansion rises and then be crushed, letting the observer feel magical and be told that the air has been purified. In fact, the crushed aerosol becomes small particles that the human eye may not be able to observe. In fact, the molecules of any nature have not changed, and the odor has not been eliminated. Therefore, people have been looking for other ways to satisfy the deodorizing effect. For example, add an M-type filter with activated carbon at the back end of an airflow path.
3. Bipolar ionizer with double needle tip (carbon fiber bundle). The generator has two high-voltage ends, DC positive and negative high voltages are applied to the each ends respectively to generate positive and negative ions, respectively. Sharp's purification ion mode would be a typical implementation. Two adjacent emitters each excite their own polar ionic air. Gas molecules or particles with the same polarity charge repel each other (formation of ion wind). If there are gas molecules or particles with opposite polarity charges in the vicinity, they will attract each other and collide with each other to become neutral molecules or particles. This process has an effect on the degradation of organic volatiles, and the energy of the gas and particles colliding with each other participate in the process of oxidative decomposition. For the floating bacteria in the air obtained during the process of mutual attraction, collision and annihilation, the electrolyte in the cell membrane is electrified and killed. For a large number of ionic gas molecules that do not have the opportunity to annihilate with the charge of the opposite polarity, they will be charged after encountering the particles, and the particles will be converted from fly ash to dust. Therefore, the performance of bipolar ionizers with opposite polarity is much better than that of mono-polar ionizers, which is determined by its own mechanism. Patent JP 2002 065838 A, filed by SHARP CORP and published on March 5^{th}, 2002, discloses an ion generator that has an electrode buried in a glass plate and another electrode provided on a surface of the glass plate. Another patent application TW 201104192 A1, filed by LUNGHWA UNIVERSITY OF SCIENCE AND TECHNOLOGY [TW] and published on February 1^{st}, 2011, discloses a high electric field plasma generator that comprises an inner electrode and a plurality of outer electrodes, which are grounded and being inferred thermally conductive. Both publications fail to disclose one or more features according to claim 1 of the current disclosure.

### SUMMARY OF THE INVENTION

An object of the present invention is to overcome the above-mentioned drawbacks of the prior art, and to provide a bipolar ion alternative generator of a porous or multi-row wire array to inject a bipolar ion gas flow in a living environment, thereby improving indoor air quality.

The technical scheme of the present invention is as follows: a bipolar ionizer for air purification, which is a plate-type structure comprising a substrate with a thermally conductive sheet, a first porous emitter plate, a second porous emitter plate, a first dielectric barrier plate, a second dielectric barrier plate, a first ground plate, and a second ground plate; wherein a first porous emitter plate disposed on a top surface of the substrate and a second porous emitter plate disposed on a bottom surface of the substrate; a first dielectric barrier plate disposed above the first porous emitter plate, and a second dielectric barrier plate disposed under the second porous emitter plate; a first ground plate disposed above the first dielectric barrier plate and the second ground plate disposed under the second dielectric barrier plate; the first porous emitter plate and the second porous emitter plate are both porous metal sheets; the first ground plate and the second ground plate are both porous metal sheets and have ion extraction mechanism; an electric field gradient formed between thermally conductive sheet and the first porous emitter plate is smaller than an electric field gradient formed between the first porous emitter plate and the first ground plate, and an electric field gradient formed between thermally conductive sheet and the second porous emitter plate is smaller than an electric field gradient formed between the second porous emitter plate and the second ground plate.

In the bipolar ionizer for air purification, thermally conductive sheet is located at the center of the substrate, and it uniformly divides the substrate into an upper substrate 31 and a lower substrate 32; and the first dielectric barrier plate and the second dielectric barrier plate are of the same material and have the same thickness.

In the bipolar ionizer for air purification, the first ground plate and the second ground plate are porous metal sheets of the same structure, and they have meshes regularly distributed in arrays.

In the bipolar ionizer for air purification, the first dielectric barrier plate and the second dielectric barrier plate are made of either high silica glass plates or modified ceramic plates.

In the bipolar ionizer for air purification, thermally conductive sheet is an electric heating sheet.

In the bipolar ionizer for air purification, the periphery of the plate-type structure is provided with a sealing ring.

A diffuser using a bipolar ionizer has a plurality of blades, on which a plurality of the above-described bipolar ionizers is disposed in the same direction.

According to the above technical solution, the ground plate and the emitter plate with the ion extraction structure form an electric field with a precise size, and the electrons of the emitter plate are led out of the dielectric barrier plate on a grounding side of the electric field. A part of the electrons meets the ground plate and flows into the ground plate to form a current, and a part of the electrons escapes from the surface of the dielectric barrier plate and meets the indoor air molecules. When the emitted electrons reach a certain rate, the oxygen molecules can be excited, converting them to ionic state, and the air quality is improved. When AC high-voltage current is input, the bipolar ions are generated alternately, so that a bipolar ion gas flow can be injected into the air to effectively improve the air quality.

### BRIEF DESCRIPTION OF THE DRAWINGS

Figure 1 is a front view of a bipolar ionizer for air purification of the present invention;
Figure 2 is a cross-sectional view of a portion A-A shown in Figure 1;
Figure 3 is a cross-sectional view of a portion B-B shown in Figure 2; and
Figure 4 is a schematic diagram of a diffuser using a bipolar ionizer.

### DETAILED DESCRIPTION OF THE INVENTION

The present invention will be further described in detail below with reference to the embodiments and the accompanying drawings.

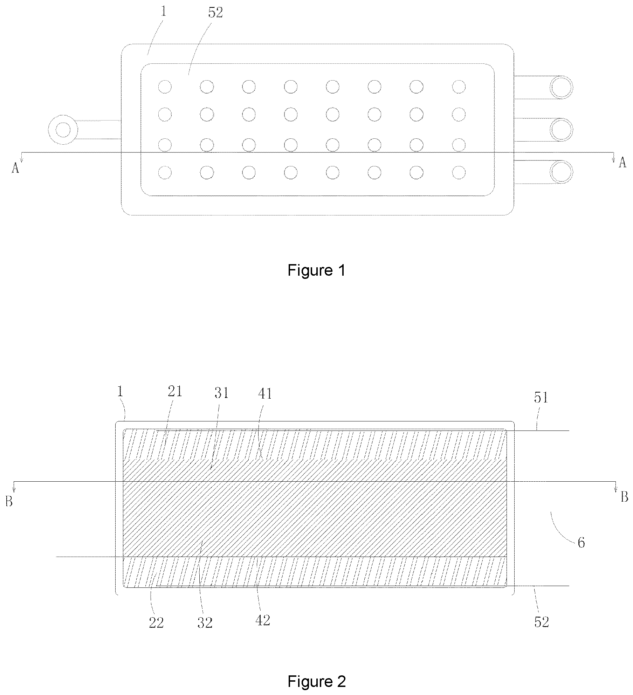

Figures 1, 2 and 3 illustrate an embodiment of a bipolar ionizer for air purification of the present invention. A thermally conductive sheet 6 is located at a center of a substrate, and the thermally conductive sheet 6 uniformly divides the substrate into an upper substrate 31 and a lower substrate 32; a first porous emitter plate 41 disposed on a top surface of the upper substrate 31 and a second porous emitter plate 42 disposed on a bottom surface of the lower substrate 32; a first dielectric barrier plate 21 is disposed on the first porous emitter plate 41; a second dielectric barrier plate 22 is disposed under the second porous emitter plate 42; a first ground plate 51 is disposed above the first dielectric barrier plate 21, and a second ground plate 52 is disposed under the second dielectric barrier 22; and the first dielectric barrier plate 21 and the second dielectric barrier plate 22 are of the same material and have the same thickness. The bipolar ionizer for air purification is a plate-type structure comprising a first ground plate 51, a first dielectric barrier plate 21, a first porous emitter plate, a substrate with a thermally conductive sheet 6, a second porous emitter plate, a second dielectric barrier plate 22, and a second ground plate 52, all of which are stacked together in sequence. The first porous emitter plate 41 and the second porous emitter plate 42 are both porous metal sheets; and the first ground plate 51 and the second ground plate 52 are both porous metal sheets. When the substrate and the first dielectric barrier 21 and the second dielectric barrier 22 are of the same material, the thickness of the substrate is greater than that of the first dielectric barrier 21 and the second dielectric barrier 22, so that the electric field gradient formed between the thermally conductive sheet 6 and the first porous emitter plate 41 is smaller than that formed between the first porous emitter plate 41 and the first ground plate 51. It is ensured that the direction of electron extraction is on the side of the first ground plate 51 and the second ground plate 52, so as to ensure that when the air conditioning system refrigerates and when possible condensation formed when its airflow passes through it, the thermally conductive sheet 6 in the substrate can ensure that electrons escaping from the dielectric barrier material are less disturbed by water molecules and can excite oxygen molecules in the air in their proper energy states.

In this embodiment, the first ground plate 51 and the second ground plate 52 are porous metal sheets having the same structure, and the meshes thereof are regularly arranged in arrays.

In this embodiment, the first dielectric barrier plate 21 and the second dielectric barrier plate 22 are made of either high silica glass plates or modified ceramic plates.

In this embodiment, the thermally conductive sheet 6 is an electric heating sheet.

In this embodiment, the periphery of the plate-type structure is provided with a sealing ring 1.

In the bipolar ionizer of the present invention, since the airflows flowing out from the ionization regions of opposite polarities attract each other, the effect of capturing smaller inhalable particles in the air is better than that of the mono-polar ionized gas, and the effect of capturing inhalable particles of smaller size is more obvious. In the present invention, an ion flow that flows out by the alternating excitation function of the bipolar ions itself has an expansion effect, and the mutual attraction mechanism of the opposite polarity ions causes the ions of opposite polarities to collide sharply outside the outlet. If no gaseous molecules of organic volatiles are encountered in the process, the two are mutually annihilated and returned to the original neutral oxygen molecule state. If it encounters the gaseous molecules of the treated organic volatiles, it is higher than the momentum of the oxygen molecules and the momentum of the two ions, effectively degrades the gaseous molecules of the organic volatiles, thereby changing its chemical properties. Usually these gaseous molecules will eventually change to gaseous molecules of water and carbon dioxide. Experiments have shown that NS-DBD with bipolar ion alternating excitation function has obvious degradation effect on formaldehyde without any filter and dust collector, and it is also easier to solve the degradation of ammonia/benzene gaseous molecules. The bipolar ionizer of the present invention is effective in killing bacteria in a very short time.

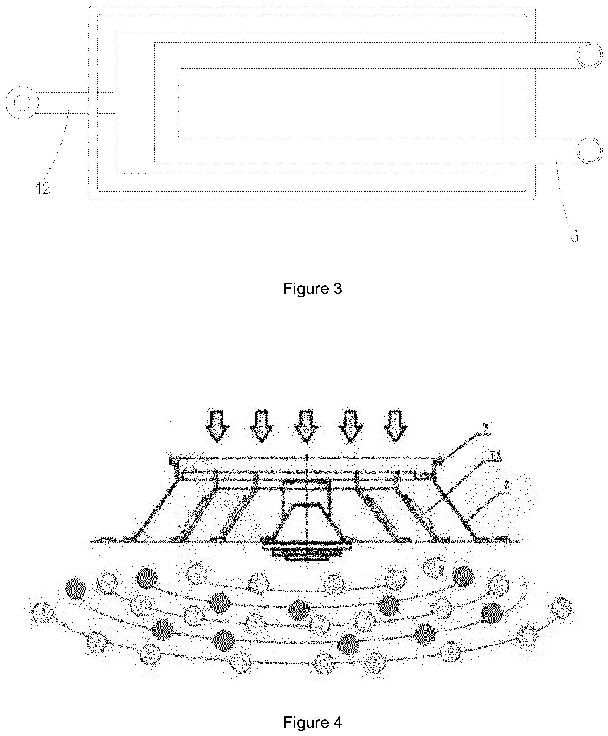

Figure 4 shows a diffuser using a bipolar ionizer. A plurality of bipolar ionizers 8 are arranged in a same direction on a number of blades 71 of the diffuser 7 and inside an air outlet. The material of the air outlet is preferably made of non-metal material, so that more airflow with positive and negative ions can be injected into the indoor space. Bipolar ions have a higher chance of encountering inhalable particles, gaseous molecules of organic volatiles, floating bacteria in indoor space than they do in net influent air flux. In this sense, the bipolar ionizer is an active purification device.

Though various embodiments of the present invention have been illustrated above, a person of the art will understand that, variations and improvements made upon the illustrative embodiments may fall within the scope of the present invention, which is defined in the appended claims.

## Claims

1. A bipolar ionizer for air purification, which is a plate-type structure comprising:
a substrate with a thermally conductive sheet (6),
a first porous emitter plate (41),
a second porous emitter plate (42),
a first dielectric barrier plate (21),
a second dielectric barrier plate (22),
a first ground plate (51), and
a second ground plate (52); wherein
the fist porous emitter plate (41) is disposed on a top surface of the substrate and the second porous emitter plate (42) is disposed on a bottom surface of the substrate the first dielectric barrier plate (21) is disposed above the first porous emitter plate (41) and the second dielectric barrier plate (22) is disposed under the second porous emitter plate (42);
the first ground plate (51) is disposed above the first dielectric barrier plate (21) and the second ground plate (52) is disposed under the second dielectric barrier plate (22)
the first porous emitter plate (41) and the second porous emitter plate (42) are both porous metal sheets;
the first ground plate (51) and the second ground plate (52) are both porous metal sheets;
an electric field gradient formed between the thermally conductive sheet (6) and the first porous emitter plate (41) is smaller than an electric field gradient formed between the first porous emitter plate (41) and the first ground plate (51), and
an electric field gradient formed between the thermally conductive sheet (6) and the second porous emitter plate (42) is smaller than an electric field gradient formed between the second porous emitter plate (42) and the second ground plate (52).

2. The bipolar ionizer for air purification as recited in claim 1, wherein the thermally conductive sheet (6) is located at a center of the substrate, and it uniformly divides the substrate into an upper substrate (31) and a lower substrate (32); and the first dielectric barrier plate (21) and the second dielectric barrier plate are of the same material and have the same thickness.

3. The bipolar ionizer for air purification as recited in claim 2, wherein both the first ground plate (51) and the second ground plate (52) have meshes regularly distributed in arrays.

4. The bipolar ionizer for air purification as recited in claim 2, wherein the first dielectric barrier plate (21) and the second dielectric barrier plate (22) are made of either high silica glass plates or modified ceramic plates.

5. The bipolar ionizer for air purification as recited in
claim 3, wherein the first dielectric barrier plate (21) and the second dielectric barrier plate (22) are made of either high silica glass plates or modified ceramic plates.

6. The bipolar ionizer for air purification as recited in claim 2, wherein the thermally conductive sheet (6) is an electric heating sheet.

7. The bipolar ionizer for air purification as recited in claim 3, wherein the thermally conductive sheet (6) is an electric heating sheet.

8. The bipolar ionizer for air purification as recited in claim 7, wherein a periphery of the plate-type structure is provided with a sealing ring (1).

9. A diffuser (7) comprising a plurality of blades (71) and a plurality of bipolar ionizers (8) as recited in claim 1, wherein the plurality of the bipolar ionizers is respectively arranged in the same direction on said plurality of blades.

## Patentansprüche

1. Bipolarer Ionisator zur Luftreinigung, der eine Struktur vom Plattentyp ist, umfassend:
ein Substrat mit einer wärmeleitenden Schicht (6),
eine erste poröse Emitterplatte (41),
eine zweite poröse Emitterplatte (42),
eine erste als dielektrische Barriere fungierende Platte (21),
eine zweite als dielektrische Barriere fungierende Platte (22),
eine erste Grundplatte (51) und
eine zweite Grundplatte (52); wobei
die erste poröse Emitterplatte (41) auf einer oberen Fläche des Substrats angeordnet ist und die zweite poröse Emitterplatte (42) auf einer unteren Fläche des Substrats angeordnet ist;
die erste als dielektrische Barriere fungierende Platte (21) über der ersten porösen Emitterplatte (41) angeordnet ist und die zweite als dielektrische Barriere fungierende Platte (22) unter der zweiten porösen Emitterplatte (42) angeordnet ist;
die erste Grundplatte (51) über der ersten als dielektrische Barriere fungierenden Platte (21) angeordnet ist und die zweite Grundplatte (52) unter der zweiten als dielektrische Barriere fungierenden Platte (22) angeordnet ist;
die erste poröse Emitterplatte (41) und die zweite poröse Emitterplatte (42) beide poröse Bleche sind;
die erste Grundplatte (51) und die zweite Grundplatte (52) beide poröse Bleche sind;
ein zwischen der wärmeleitenden Schicht (6) und der ersten porösen Emitterplatte (41) ausgebildeter Gradient des elektrischen Feldes kleiner ist als ein zwischen der ersten porösen Emitterplatte (41) und der ersten Grundplatte (51) ausgebildeter Gradient des elektrischen Feldes und
ein zwischen der wärmeleitenden Schicht (6) und der zweiten porösen Emitterplatte (42) ausgebildeter Gradient des elektrischen Feldes kleiner ist als ein zwischen der zweiten porösen Emitterplatte (42) und der zweiten Grundplatte (52) ausgebildeter Gradient des elektrischen Feldes.

2. Bipolarer Ionisator zur Luftreinigung nach Anspruch 1, wobei die wärmeleitende Schicht (6) sich in einer Mitte des Substrats befindet und das Substrat gleichmäßig in ein oberes Substrat (31) und ein unteres Substrat (32) unterteilt; und die erste als dielektrische Barriere fungierende Platte (21) und die zweite als dielektrische Barriere fungierende Platte aus dem gleichen Material sind und die gleiche Dicke aufweisen.

3. Bipolarer Ionisator zur Luftreinigung nach Anspruch 2, wobei sowohl die erste Grundplatte (51) als auch die zweite Grundplatte (52) gleichmäßig in Anordnungen verteilte Maschen aufweisen.

4. Bipolarer Ionisator zur Luftreinigung nach Anspruch 2, wobei die erste als dielektrische Barriere fungierende Platte (21) und die zweite als dielektrische Barriere fungierende Platte (22) entweder aus Platten aus siliciumdioxidreichem Glas oder aus Platten aus modifizierter Keramik hergestellt sind.

5. Bipolarer Ionisator zur Luftreinigung nach Anspruch 3, wobei die erste als dielektrische Barriere fungierende Platte (21) und die zweite als dielektrische Barriere fungierende Platte (22) entweder aus Platten aus siliciumdioxidreichem Glas oder aus Platten aus modifizierter Keramik hergestellt sind.

6. Bipolarer Ionisator zur Luftreinigung nach Anspruch 2, wobei die wärmeleitende Schicht (6) eine elektrische Heizschicht ist.

7. Bipolarer Ionisator zur Luftreinigung nach Anspruch 3, wobei die wärmeleitende Schicht (6) eine elektrische Heizschicht ist.

8. Bipolarer Ionisator zur Luftreinigung nach Anspruch 7, wobei ein Rand der Struktur vom Plattentyp mit einem Dichtungsring (1) versehen ist.

9. Diffusor (7), der eine Mehrzahl von Lamellen (71) und eine Mehrzahl bipolarer Ionisatoren (8) nach Anspruch 1 umfasst, wobei die Mehrzahl bipolarer Ionisatoren jeweils in der gleichen Richtung auf der Mehrzahl von Lamellen angeordnet ist.

## Revendications

1. Ionisant bipolaire pour purification de l'air, qui est une structure du type à plaques comprenant :
un substrat présentant une feuille thermoconductrice (6),
une première plaque émettrice poreuse (41),
une deuxième plaque émettrice poreuse (42),
une première plaque barrière diélectrique (21),
une deuxième plaque barrière diélectrique (22),
une première plaque de terre (51) et
une deuxième plaque de terre (52) ; où
la première plaque émettrice poreuse (41) est disposée sur une surface supérieure du substrat et la deuxième plaque émettrice poreuse (42) est disposée sur une surface inférieure du substrat ;
la première plaque barrière diélectrique (21) est disposée en dessus de la première plaque émettrice poreuse (41) et la deuxième plaque barrière diélectrique (22) est disposée en dessous de la deuxième plaque émettrice poreuse (42) ;
la première plaque de terre (51) est disposée en dessus de la première plaque barrière diélectrique (21) et la deuxième plaque de terre (52) est disposée en dessous de la deuxième plaque barrière diélectrique (22) ;
la première plaque émettrice poreuse (41) et la deuxième plaque émettrice poreuse (42) sont toutes deux des feuilles métalliques poreuses ;
la première plaque de terre (51) et la deuxième plaque de terre (52) sont toutes deux des feuilles métalliques poreuses ;
un gradient de champ électrique formé entre la feuille thermoconductrice (6) et la première plaque émettrice poreuse (41) est plus faible qu'un gradient de champ électrique formé entre la première plaque émettrice poreuse (41) et la première plaque de terre (51) et
un gradient de champ électrique formé entre la feuille thermoconductrice (6) et la deuxième plaque émettrice poreuse (42) est plus faible qu'un gradient de champ électrique formé entre la deuxième plaque émettrice poreuse (42) et la deuxième plaque de terre (52).

2. Ionisant bipolaire pour purification de l'air tel que décrit à la revendication 1, où la feuille thermoconductrice (6) est située à un centre du substrat et divise uniformément le substrat en un substrat supérieur (31) et un substrat inférieur (32) ; et où la première plaque barrière diélectrique (21) et la deuxième plaque barrière diélectrique sont constituées du même matériau et ont la même épaisseur.

3. Ionisant bipolaire pour purification de l'air tel que décrit à la revendication 2, où la première plaque de terre (51) et la deuxième plaque de terre (52) présentent toutes deux des mailles distribuées régulièrement en réseaux.

4. Ionisant bipolaire pour purification de l'air tel que décrit à la revendication 2, où la première plaque barrière diélectrique (21) et la deuxième plaque barrière diélectrique (22) sont constituées soit de plaques de verre à haute teneur en silice, soit de plaques d'une céramique modifiée.

5. Ionisant bipolaire pour purification de l'air tel que décrit à la revendication 3, où la première plaque barrière diélectrique (21) et la deuxième plaque barrière diélectrique (22) sont constituées soit de plaques de verre à haute teneur en silice, soit de plaques d'une céramique modifiée.

6. Ionisant bipolaire pour purification de l'air tel que décrit à la revendication 2, où la feuille thermoconductrice (6) est une feuille chauffante électrique.

7. Ionisant bipolaire pour purification de l'air tel que décrit à la revendication 3, où la feuille thermoconductrice (6) est une feuille chauffante électrique.

8. Ionisant bipolaire pour purification de l'air tel que décrit à la revendication 7, où une périphérie de la structure de type à plaques est dotée d'un anneau d'étanchéité (1).

9. Diffuseur (7) comprenant une pluralité de pales (71) et une pluralité d'ionisants bipolaires (8) tels que décrits à la revendication 1, où la pluralité d'ionisants bipolaires sont respectivement agencés dans la même direction sur ladite pluralité de pales.
